(19) Europäisches Patentamt

European Patent Office

Office européen des brevets

(11) **EP 4 755 304 A1**

(12) **EUROPEAN PATENT APPLICATION**

(43) Date of publication:
**10.06.2026 Bulletin 2026/24**

(21) Application number: **24217968.7**

(22) Date of filing: **06.12.2024**

(51) International Patent Classification (IPC):
**A61B 6/00** *(2024.01)*    **A61B 6/40** *(2024.01)*
**A61B 6/42** *(2024.01)*    **A61B 6/50** *(2024.01)*

(52) Cooperative Patent Classification (CPC):
**A61B 6/482; A61B 6/032; A61B 6/5217;**
**G01N 23/00;** A61B 6/405; A61B 6/4241;
A61B 6/505

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AE Eindhoven (NL)**

(72) Inventors:
• **DAERR, Heiner**
  **Eindhoven (NL)**
• **BRENDEL, Bernhard Johannes**
  **Eindhoven (NL)**

(74) Representative: **Philips Intellectual Property &**
**Standards**
**High Tech Campus 52**
**5656 AG Eindhoven (NL)**

(54) **SPECTRAL CT IMAGING**

(57)     Proposed concepts thus aim to provide a method for determining the decomposition matrix of a pair of CT images. In particular, embodiments aim to provide a method for determining the decomposition matrix based on values of pixels in each image of the pair of CT images that are determined to depict bone tissue of the subject.

```
┌─────────────────────────────────────────┐
│         DETERMINE FIRST PIXEL VALUE      │ ─110
└─────────────────────────────────────────┘
                    │
                    ▼
┌─────────────────────────────────────────┐
│        DETERMINE SECOND PIXEL VALUE      │ ─120
└─────────────────────────────────────────┘
                    │
                    ▼
┌─────────────────────────────────────────┐
│       DETERMINE DECOMPOSITION MATRIX     │ ─130
│  ┌───────────────────────────────────┐  │
│  │ DETERMINE CANDIDATE DECOMPOSITION │  │ ─132
│  │             MATRIX                │  │
│  └───────────────────────────────────┘  │
│  ┌───────────────────────────────────┐  │
│  │ PERFORM MATERIAL DECOMPOSITION OF │  │ ─134
│  │   FIRST AND SECOND PIXEL VLAUES   │  │
│  └───────────────────────────────────┘  │
│  ┌───────────────────────────────────┐  │
│  │ COMPARE SET OF PREDICTED          │  │ ─136
│  │ COORDINATED TO PREDETERMINED LINE │  │
│  │         IN MATERIAL SPACE         │  │
│  └───────────────────────────────────┘  │
│  ┌───────────────────────────────────┐  │
│  │ DETERMINE AN ADJUSTMENT TO BE TO  │  │ ─138
│  │ THE CANDIDATE DECOMPOSITION MATRIX│  │
│  └───────────────────────────────────┘  │
└─────────────────────────────────────────┘
                                    ─100
```

**FIG. 1**

EP 4 755 304 A1

**Description**

FIELD OF THE INVENTION

This invention relates to the field of medical imaging, and in particular to spectral CT imaging.

BACKGROUND OF THE INVENTION

Spectral CT imaging (or dual-energy CT imaging, DECT) is an imaging technique that provides additional clinical and diagnostic value compared to single-energy conventional CT imaging (SECT). It typically involves obtaining at least two sets of CT imaging data using radiation of at least two different energy spectra. Dual-energy CT imaging may be achieved using dedicated spectral CT scanners such as dual-layer, fast kVp-switching, dual-source, and photon counting CT systems. Alternatively, dual-energy CT imaging can be done using conventional CT systems if the field-of view is scanned with two different kVp settings of the x-ray tube of the CT scanner in two separate scans to produce two images of the same field of view at different effective x-ray energies. More advanced techniques such as faster kVp switching or interscan kVp-switching may also be used.

The advantage of spectral or dual-energy CT imaging is that it allows for material decomposition of the CT images which provides information on the composition of the biological and inorganic material in the field of view of the CT scan. Since the attenuation coefficients of materials vary with energy, spectral information obtained by imaging the patient at least two different energies, allows for the attenuation coefficients associated with the various regions of the image to be derived - from which the composition (i.e. the percentage composition of bone, fat, and other biological tissues) can be deduced.

Performing material decomposition of the images may not however be straightforward as the necessary information for transforming the image to material space (which typically may require knowledge of the effective energies of the two images) may not be well known.

SUMMARY OF THE INVENTION

The invention is defined by the claims.

According to an aspect of the invention, there is provided a computer-implemented method for determining a decomposition matrix of a pair of CT images of a subject, each image of the pair of CT images being associated with a different energy spectrum (called pair of dual-energy CT images).

The method comprises: determining a first pixel value of a first image of the pair of CT images, the first pixel value describing the value of a first pixel of the first image that depicts bone tissue of the subject; determining a second pixel value of the second image of the pair of CT images, the second pixel value describing the value of a second pixel of the second image that depicts bone tissue of the subject, wherein the first and second pixel depict the same region of bone tissue of the subject; and determining, based on the determined first and second pixel values, a decomposition matrix of the pair of CT images for performing a material decomposition of the pair of CT images.

Proposed concepts thus aim to provide schemes, solutions concepts, designs, methods and systems pertaining to determining the decomposition matrix of a pair of CT images. In particular, embodiments aim to provide a method for determining the decomposition matrix based on values of pixels in each image of the pair of CT images that are determined to depict bone tissue of the subject.

CT imaging typically involves the use of rotating x-ray tubes and an associated set of x-ray detectors to measure the attenuation in the x-ray radiation caused by tissues inside the body. The attenuation of the x-ray signals detected by the CT scanner may vary significantly depending on the spectrum of the incident x-ray radiation and the composition of the material the radiation passes through. Material decomposition of CT images involves the translation of the measured attenuation in the x-ray signal to a description of the material composition of the region of the imaged subject depicted in the CT image. Since the measured signal is affected by both the composition of the material the detected x-rays have passed through and the energy of the incident x-ray radiation, material decomposition requires the subject to be imaged with at least two different incident energy spectra such that these two effects may be decoupled. The variation between the measured attenuated signals associated with the different incident energy spectra may therefore be used to gather information about the material composition of the tissues in the field of view of the CT image. In practice, material decomposition typically involves the application of a decomposition matrix to the pixel values of a pair of CT images to transform the images to a two-dimensional material space.

Accurate material decomposition of CT images it is typically achieved using accurate estimate of the effective energy of the images. This may be different from the energy spectrum of the x-ray radiation emitted by the x-ray tube of the CT scanner due to beam hardening effects (beam hardening relates to the phenomenon that lower energy photons will suffer greater attenuation than high energy photons and therefore the subject effectively acts as a high-pass filter) which will also vary with the composition of the imaged region and with scan position. Therefore, in order to perform a material decomposition of a CT image it is often necessary to gather detailed information about the parameters of the CT scan set-up and the subject being scanned (which may not always be available) or perform long and tedious calibration procedures before every scan.

It was realized that pixel values of regions of CT images at two different energy spectra that depict the same region of the subject's bone tissue carry sufficient information that the decomposition matrix for a pair of CT images may be determined based on the value of only these pixels. Bone tissue is present in almost all body regions typically imaged by CT scanners, does not deform between scans, and has an attenuation value that varies significantly with different incident energy spectra in a predictable way. Therefore, analyzing the variation in the attenuation of x-ray signals of at least two different energies identified to be caused by a region of bone tissue of the subject, may allow for accurate determination of the material decomposition matrix for the image which may then be used to perform a material decomposition of the CT images. Other tissues may be unsuitable for this application since water-like tissues such as blood, organs, or muscles do not change sufficiently with different incident energy spectra and fatty tissue is not prevalent in all body regions (such as the head or hand) and in all patients.

The proposed invention is therefore advantageous in that it allows for the material decomposition of CT images from their pixel values alone, without the need for complicated information about the imaging set-up or for calibration procedures to be carried out.

Ultimately, an improved method for determining a decomposition matrix of a pair of CT images obtained via a dual-energy CT imaging procedure may be supported by the proposed concept(s).

In some embodiments, determining the decomposition matrix of the pair of CT images may comprise determining a candidate decomposition matrix of the pair of CT images; performing, based on the determined candidate decomposition matrix, a material decomposition of the first and second pixel values to determine a predicted composition of the bone tissue of the subject; determining an adjustment to be made to the candidate decomposition matrix based on the predicted composition of the bone tissue of the subject; and determining the decomposition matrix of the pair of CT images based on the determined adjustment. The value of the material decomposition of pixels depicting bone tissue may be expected to have certain predetermined values. Therefore, by testing the result of applying various candidate decomposition matrices to the first and second pixel values, it may be determined how close the candidate decomposition matrix is to the decomposition matrix required for performing material decomposition of the pair of CT images. This may be used to produce an accurate and informed estimate of the decomposition matrix of the two CT images.

In some embodiments, the predicted composition of the bone tissue of the subject may comprise a set of predicted coordinates of the bone tissue in material space. Determining the decomposition matrix of the pair of CT images may then further comprise: comparing the set of predicted coordinates of the bone tissue in the material space to a predetermined line in the material space; and determining the decomposition matrix of the pair of CT images based on this comparison. It may be known that regions with high bone tissue composition are expected to lie on a predetermined line in material space. Using this line to assess the suitability of a candidate decomposition matrix for the pair of CT images may therefore allow for the estimate of the decomposition matrix to be formed from the analysis of only the pixels depicting bone tissue.

In some embodiments, determining an anatomical feature associated with pixels of the first and second images associated with the first and second pixels; and selecting the predetermined line in material space based on the determined anatomical feature. The bone tissue in different regions of a patient's body may be expected to have slightly different compositions. In addition, also age, sex and pre-existing condition may be considered as well for selecting a bone line in the material space. Therefore, by using a specific line in material space for the analysis of the first and second pixels, a more accurate assessment of the material decomposition of the images may be achieved.

In some embodiments, the predicted coordinates of the bone tissue in material space may comprise: a Compton effect component; and a photoelectric component. Different biological materials may result in different levels of Compton scattering and photoelectric effect and therefore these may be a useful basis for describing the material composition of the bone tissue of the patient.

In some embodiments, determining the first and second pixel values may comprise: comparing the value of a first candidate pixel of the first image to a first threshold value; comparing the value of a second candidate pixel value of the second image to a second threshold value; determining a ratio of the values of the first candidate pixel and the second candidate pixel; comparing the determined ratio of the value of the first candidate pixel to the value of the second candidate pixel to a predetermined pixel ratio value; and determining the first and second pixel values based on these comparisons. Thus, the proposed invention may advantageously allow for the automatic selection of pixels in the image that depict bone tissue using pixel value thresholding - this may be more efficient than manual selection.

In some embodiments, determining the decomposition matrix of the pair of CT images may comprise comparing the first pixel value to a plurality of precalculated pixel values each comprising a predicted value of a pixel that depicts bone tissue for a CT image having a different respective effective energy; comparing the second pixel to the plurality of precalculated pixel values; and determining the decomposition matrix of the pair of CT images based on these comparisons. The use of a table of precalculated pixel values in this way may allow for easy and efficient determination of the decomposition matrix of the pair of CT images based on the values of pixels within the images that depict bone tissue.

In some embodiments, determining the decomposition matrix of the pair of CT images may comprise: determining, based on the first and first and second pixels values, an effective energy value of each of the first and second images of the pair of CT images; and determining, based on the determined effective energy value of each of the first and second images, the decomposition matrix of the pair of CT images. The effective energies can be used to calculate to decomposition matrix but may not be known for a dual-energy CT images (as this often varies per axial slice even if the images are virtually monochromatic). Determining the effective energies of the pair of CT images from the first and second pixel values may therefore allow for efficient determination of the decomposition matrix.

In some embodiments, the method may further comprise: determining a first body part of the subject depicted in the pixel of the first image of the pair of CT images associated with the first pixel value; determining a second body part of the subject depicted in the pixel of the second image of the pair of CT images associated with the second pixel value; and determining the decomposition matrix of the pair of CT images based on the first pixel values, the second pixel value, the first body part and the second body part. The attenuation coefficient of bone tissue may vary depending on which body part it is associated with and therefore incorporating this information into the determination of the decomposition matrix may result in a more accurate material decomposition of the image.

In some embodiments, the first and second body parts may comprise at least one of: a head; a thorax; an abdomen; a kidney region; a pelvis; and an extremity. These regions may be the most common regions imaged by a CT scanner and may provide suitable categorization of the images for accurate determination of the decomposition matrix of the pair of CT images.

In some embodiments, the method may further comprise performing, before the first and second pixel values are determined, an image registration process of the first and second images of the pair of CT images such that the field of view of the first image is substantially identical to the field of view of the second image. This may make ensuring that the first and second pixels depict the same region of bone tissue easier.

According to another aspect of the proposed invention, there is provided a computer program comprising computer program code means adapted, when said computer program is run on a computer, to implement the method of any of the embodiments described above.

According to yet another aspect of the proposed invention, there is provided a system for determining a decomposition matrix of a pair of CT images of a subject, each image of the pair of CT images being associated with a different energy spectrum. The system comprises a processing arrangement configured to: determine a first pixel value of a first image of the pair of CT images, the first pixel value describing the value of a first pixel of the first image that depicts bone tissue of the subject; determine a second pixel value of the second image of the pair of CT images, the second pixel value describing the value of a second pixel of the second image that depicts bone tissue of the subject, wherein the first and second pixel values depict the same region of bone tissue of the subject; and determine, based on the determined first and second pixel values, a decomposition matrix of each of the first and second images of the pair of CT images.

Embodiments may be employed in combination with conventional/existing CT imaging methods and systems. In this way, embodiments may integrate into legacy systems to improve and/or extend their functionality and capabilities. An improved CT imaging system that allows for the automatic material decomposition of dual-energy CT imaging scans of a subject may therefore be provided by proposed embodiments. Off-line or off-premise computation (cloud computing) may also be provided by proposed embodiments.

Thus, there may be proposed concepts for determining the decomposition matrix of a pair of CT images of a subject, each image of the pair of CT images being associated with a different energy spectrum. These and other aspects of the invention will be apparent from and elicited with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 depicts a simplified flow diagram of a method for determining a decomposition matrix of a pair of CT images according to a proposed embodiment;
Fig. 2 depicts a simplified diagram of the distribution of various biological tissues in material space;
Fig. 3A is a schematic graph of first pixel value against second pixel value for a pair of CT images;
Fig. 3B is a schematic graph depicting the transformed values of the first and second pixel values in material space;
Fig. 4 is a simplified flow diagram of a method for determining a decomposition matrix of a pair of CT images according to an alternative proposed embodiment;
Fig. 5 is a simplified flow diagram of a method for determining the first and second pixel values according to a proposed embodiment;

Fig. 6 is a simplified block diagram of a system for determining a decomposition matrix of a pair of CT images according to another aspect of the proposed invention; and

Fig. 7 is a simplified block diagram of a computer within which one or more parts of an embodiment may be employed.

DETAILED DESCRIPTION OF THE EMBODIMENTS

The invention will be described with reference to the Figures.

Embodiments of the invention aim to provide a concept for automatically determining the decomposition matrix associated with a pair of CT images obtained via dual-energy CT imaging techniques. This is achieved by determining the value of at least one pixel in each image that depicts a region of bone tissue and using these determined values to determine a decomposition matrix of the pair of CT images.

Material decomposition of CT images is an important technique used for clinical diagnosis and assessment. Material decomposition is typically achieved using dual energy CT (DECT) imaging techniques which involve the acquisition of CT images of a patient using at least two different incident energy spectra. Since the attenuation coefficient associated with a pixel of a CT image is both material and energy dependent, by obtaining measurements of the attention coefficient for a given field of view at two distinct energies, information about the basic components of the material depicted in the field of view may also be derived.

The principle of material decomposition involves approximating the attenuation coefficient associated with a given pixel of the obtained CT image by a linear combination of the intrinsic attenuation values of a set of basis materials. The various weightings of this linear combination may therefore approximate the relative presence of the different basis materials in the region depicted by the pixel of the CT image. By forming pairs of pixels of the pair of CT images that depict identical regions of the subject (and therefore are associated with substantially identical material compositions), the pair of CT images may be described by a set of coordinates in energy space (i.e., the attenuation coefficients associated the formed pairs of pixels) or a set of coordinates in material space (the weightings of the linear combination of material specific attenuation values). Material decomposition involves determining the transformation for transforming the coordinates of the images in energy space to a set of coordinates in material space.

The transformation process may be described by the equation,

$$\begin{pmatrix} \mu_1 \\ \mu_2 \end{pmatrix} = \begin{pmatrix} \mu^{ph}(E_1) & \mu^{sc}(E_1) \\ \mu^{ph}(E_2) & \mu^{sc}(E_2) \end{pmatrix} \begin{pmatrix} ph \\ sc \end{pmatrix} = A \begin{pmatrix} ph \\ sc \end{pmatrix}, \tag{1}$$

where $\mu_1$, and $\mu_2$ are the attenuation coefficients associated with the first and second pixel values respectively, $E_1$ and $E_2$ are the effective energies of the first and second images of the pair of CT images, and $ph$ and $sc$ are the coordinates in material space. In this example, the basis in material space is taken to be the attenuating effect of the photoelectric effect ($ph$) and the Compton effect ($sc$). Other basis materials may equally well be used, for example water and iodine (which is often used as a contrast agent in CT imaging), or bone tissue and soft tissue.

The decomposition matrix of the pair of CT images is given by the inverse of the matrix $A$ in equation 1 and is defined as the matrix describing the transformation required to translate the coordinates of the two images in energy space to the co-ordinates of the pair of images in material space. The decomposition matrix will depend on properties of the CT scanner (e.g., the energy spectrum used, the detector parameters, and any processing carried out on the images), and the composition, size, and shape of the object being imaged.

Once way in which the decomposition matrix may be calculated is by determining the effective energies of the two CT images, from which the material specific attenuation coefficients ($\mu^{ph}$ and $\mu^{sc}$) may be calculated. However, the effective energies are not always known (due to beam hardening and other effects) and therefore it may be difficult to formulate the decomposition matrix of a pair of CT images. Further, the effective energy (and hence the decomposition matrix) may vary between patients and for each image slice taken of the same patient - further increasing the complication of determining its value. Calibration procedures may typically be carried out to determine the values of the decomposition transformation, however these can be long and tedious and therefore are not recommended in clinical settings where efficiency is often key. These calibration procedures are also often subject to drifts and therefore may not be reliable over long periods of use of the dual CT scanner. Implementing a pure data driven software solution, as suggested by the proposed invention, is one approach to circumvent calibrations and improve the ease of use.

In this invention, we propose an analytic method for determining the decomposition matrix of a pair of CT images that relies only on the values of certain pixels in the CT images and does not require any system calibration or additional system or image information. The actual kVp setting information for the scan associated with the pair of CT images is not needed. Thus, the proposed approach may be applied to CT images obtained from a wide range of different CT scanner devices and can be implemented as a post-processing step.

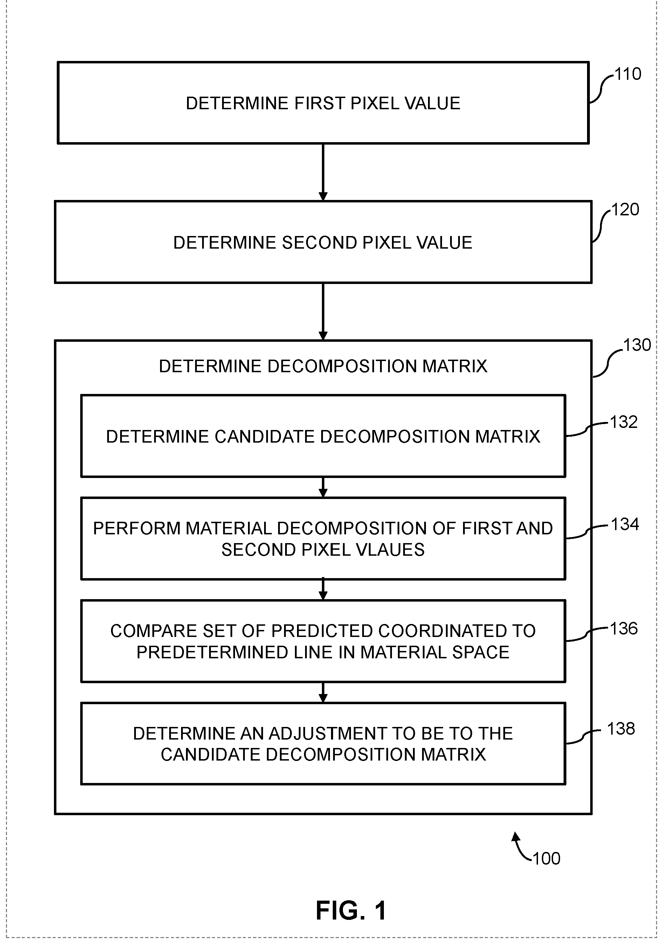

Referring now to Fig. 1, there is depicted a simplified flow diagram of a computer-implemented method 100 for determining a decomposition matrix of a pair of CT images of a subject according to a proposed embodiment.

Each of the pair of the CT images is associated with a different energy spectrum. The pair of CT images may thus have been acquired using a CT scanner capable of imaging a patient using the emission of x-rays of at least two different energy spectra. Furthermore, in this exemplary embodiment, the images are spatially aligned to each other. This is achieved by performing an image registration step so as to ensure that the field of view of the first image of the pair of CT images is substantially identical to the field of view of the second image of the pair of CT images.

The method 100 commences with a step 110 comprising determining a first pixel value of a first image of the pair of CT images, the first pixel value describing the value of a first pixel of the first image that depicts bone tissue of the subject. In this exemplary embodiment, a clinician is presented with the first image of the pair of CT images and manually identifies within the image a region that depicts bone tissue. A pixel within this region of the image is then selected and its value determined.

The method 100 then proceeds to step 120 comprising determining a second pixel value of the second image of the pair of CT images, the second pixel value describing the value of a second pixel of the second image that depicts bone tissue of the subject. The first pixel and the same region of bone tissue of the subject. In this embodiment, since the first and second images are spatially aligned the second pixel is selected to be the pixel at the same coordinate within the second image as the first pixel is within the first image of the pair of CT images.

Once the first and second pixel values have been determined, the method proceeds to a step 130 comprising determining, based on the first and second pixel values, a decomposition matrix of the pair of CT images for performing a material decomposition of the pair of CT images. In this exemplary embodiment, step 130 comprises the sub-steps 132, 134, 136, and 138.

Step 132 comprises determining a candidate decomposition matrix of the pair of CT images.

Step 134 comprises performing, based on the determined candidate decomposition matrix, a material decomposition of the first and second pixel values to determine a predicted composition of the bone tissue of the subject.

Step 136 comprises determining an adjustment to be made to the candidate decomposition matrix based on the predicted composition of the bone tissue of the subject.

Step 138 comprises determining the decomposition matrix based on the determined adjustment to be made to the candidate decomposition matrix.

The candidate decomposition matrix may in some embodiments be selected based on known information about the CT imaging procedure that has been carried out. For example, information on the scanner set-up parameters, the patient, and the body part being imaged may be used to preselect a candidate decomposition matrix that is known may approximate the decomposition matrix of the pair of CT images. However, this information is not required for the selection of a candidate decomposition matrix and the same candidate decomposition matrix may be used regardless of the properties of the CT imaging procedure being carried out. This may be advantageous in that no information other than the pixel values of the obtained image may be required for determining the material decomposition of the image.

In this exemplary embodiment the predicted composition of the bone tissue of the subject comprises a set of predicted 2D co-ordinates associated with the selected pixels of the first and second images of the pair of CT images in a 2D material space. These co-ordinates may be calculated with respect to any suitable basis in material space. For example, the predicted coordinates may comprise: a Compton effect component; and a photoelectric effect component.

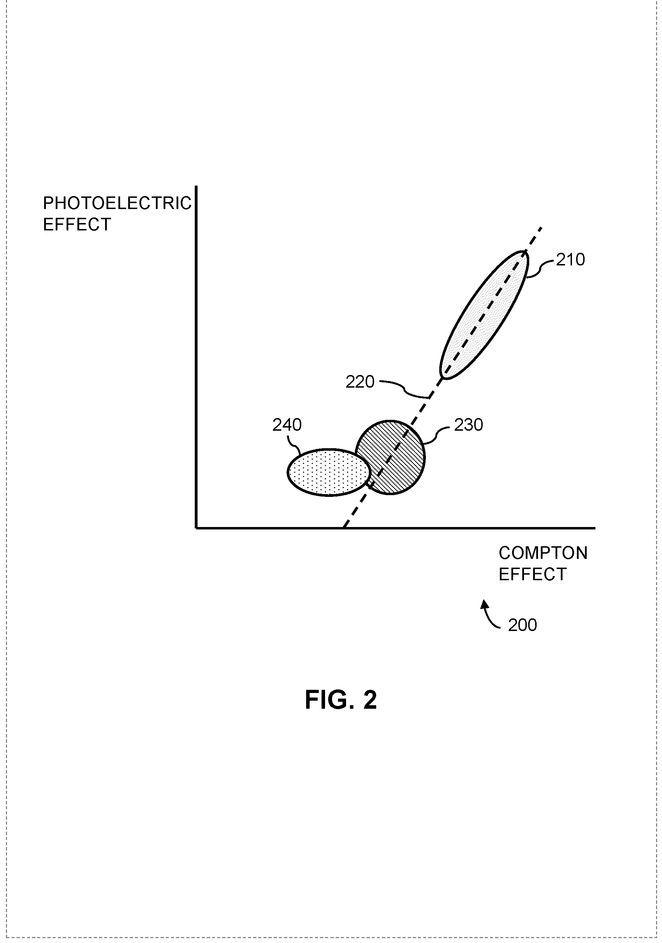

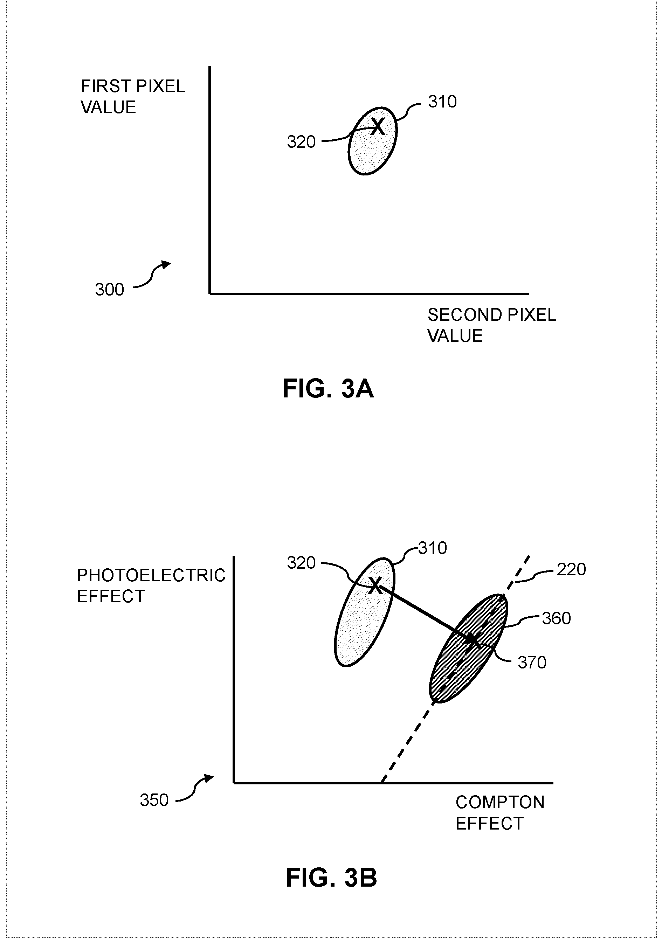

There are various ways in which the suggested adjustment to the candidate decomposition may be calculated. One example of such a method is now discussed with reference to Figs 2, 3A, and 3B.

Fig. 2 depicts a simplified diagram of the distribution of various biological tissues in material space.

The graph 200 of Fig. 2, depicts a plot of the photoelectric effect component against the Compton effect component for various biological materials that may be depicted in a CT image of a patient. The grey shaded region 210 corresponds to the region of the graph 200 in which bone tissue is likely to lie. As can be seen clearly from the Figure, these values are expected to lie close to a line 220 referred to as a bone line in material space. In contrast, soft tissue (represented by the dashed region 230) and fat tissue (represented by the dotted region 240) show no clear linear relation in material space.

It has been realized that due to the fact that bone tissue may have an approximately linear relationship in material space, the values of the bone pixel values for a pair of CT images may contain information that can be used for the determination of the decomposition matrix for the pair of images.

Figs 3A and 3B show an example of how the principle of a bone line in material space may be used for the determination of the decomposition matrix of a pair of CT images.

Thus, in an exemplary embodiment, the predicted composition of the bone tissue of the subject comprises a set of predicted coordinates of the bone tissue in material space and determining the adjustment to be made to the candidate decomposition matrix based on the predicted composition of the bone tissue of the subject comprises comparing the set of predicted coordinates of the bone tissue in the material space to a predetermined line in material space.

A pair of CT images are obtained of a patient, each of the pair of CT images having been acquired with radiation of a different energy spectrum. At least one pixel in each of the images is identified as depicting bone tissue. This may be done manually or automatically. In some embodiments, before the regions depicting bone tissue are identified an image registration process may be carried out in order to ensure the first and second image of the pair of CT images have the same field of view and are spatially aligned.

Typically, the pair of images in a dual-CT imaging procedure are obtained using two separate scans with two different kVp settings, which may result in a small but unneglectable motion between the two scans. Image registration involves spatial alignment of the two images until they have matching coordinate systems. Therefore, if a pixel is identified as depicting bone tissue in the first image, the corresponding pixel of the second image at the same position in the image should depict the same region of bone tissue and therefore be associated with a substantially identical material composition. However, image registration is not required for the purposes of the invention. It may be possible in some embodiments for a pair of pixels (each of the pair of pixels associated with a different image of a pair of obtained dual energy CT images) depicting a substantially identical region of bone tissue to be identified without the pair of CT images being spatially aligned. This may be achieved via manual selection of the first and second pixels, or automatic detection using an object detection model for example.

The determined pixel values of the pairs of pixel(s) of the first and second images of the pair of CT images that depict bone are shown in the graph 300 of Fig. 3A. A single pair pixel values of the pair of images may be identified (represented by the cross 310) or a plurality of pixel values may be determined for each of the two images (represented by the shaded region 320) resulting in a plurality of pairs of pixel values. Each pair of pixel values is associated with a single region of bone tissue of the subject, by which it is meant that each pixel of a given pair is associated with a substantially identical field of view.

A candidate decomposition matrix is then used to perform a material decomposition of the identified bone pixel values of the pair of CT images to determine a predicted composition of the bone tissue of the subject comprising a set of coordinates in a 2D material space. Thus, the region 310 and the point 320 may be represented in a 2D material space as shown in the graph 350 of Fig. 3B.

The graph 350 further contains a line 220 comprising a predetermined line in material space along which it is expected pixels depicting bone tissue to lie. Determining the decomposition matrix of the pair of CT images may comprise determining an adjustment to the candidate decomposition matrix in order to shift the transformed co-ordinates of the region 310 to a region 360 representing the best fit region of the transformed co-ordinates around the bone line 220. In the case, where only a single pixel value is identified in each image of the pair of CT images, once the candidate transformed coordinates of the point 320 have been calculated using the candidate decomposition matrix, the closest point on the line 220 to the candidate transformed coordinates 370 may be identified. The adjustment to the candidate decomposition matrix required to shift the point 320 to the point 370 may then be calculated.

The determination of the decomposition matrix may therefore involve the minimization of a cost function given by,

$$\min_{E_1, E_2} \sum_i \left\| \begin{pmatrix} \mu^{ph}(E_1) & \mu^{sc}(E_1) \\ \mu^{ph}(E_2) & \mu^{sc}(E_2) \end{pmatrix}^{-1} \begin{pmatrix} \mu^{bone}(p_i) \\ \mu^{bone}(p_i) \end{pmatrix} - a_i \begin{pmatrix} t_{sc}^{bone} \\ t_{ph}^{bone} \end{pmatrix} \right\|^2 \qquad (2)$$

where $\left( t_{sc}^{bone}, t_{ph}^{bone} \right)$ describes the coordinates of the bone line 220 and $p_i$ are the values of the identified pixels in the first and second images of the pair of CT images that depict bone tissue of the subject.

Bone tissue associated with different regions of the body may have different compositions. Therefore, in some embodiments, the position of the bone line 220 may be pre-selected using known information about the composition of different types of bone tissue. For example, the proposed method for determining the decomposition matrix of a pair of CT images may comprise determining an anatomical feature associated with pixels of the first and second images associated with the first and second pixel values; and selecting a predetermined line in material space (the bone line 220) based on the determined anatomical feature. Thus, information about the body regions depicted in the first and second images may in some embodiments be used to select the position of the bone line in material space which may result in a more accurate determination of the decomposition matrix of the pair of CT images.

The discussion of Figs 1-3 above describes just one way in which the decomposition matrix of a pair of CT images may be determined based on a first pixel value and a second pixel value of first and second images of the pair of CT images, respectively. Other embodiments may employ alternative methods. For example, instead of assessing the decomposition matrix by analyzing the values of regions of bone tissue in a pair of CT images in material space, the calculation may be done in CT image space.

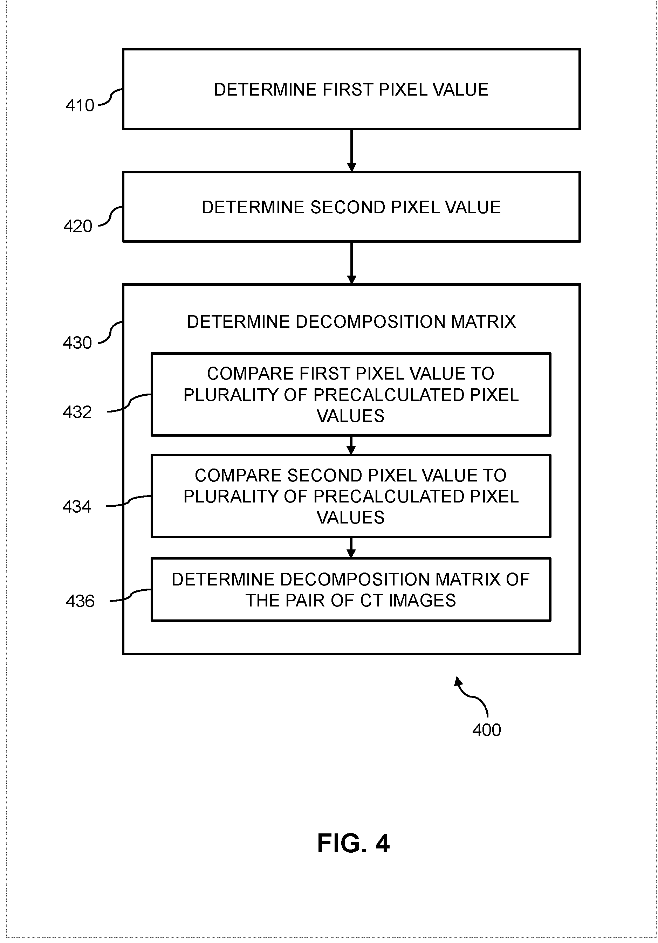

Referring now to Fig. 4, there is depicted a simplified flow diagram of a method 400 for determining the decomposition matrix of a pair of CT images of a subject according to an alternative proposed embodiment. As in the method 100, each image of the pair of CT images is associated with a different energy spectrum, i.e., the pair of CT images has been acquired using a dual-energy CT imaging procedure.

The method 400 commences with a step 410 comprising determining a first pixel value of a first image of the pair of CT images, the first pixel value describing the value of a first pixel of the first image that depicts bone tissue of the subject.

Step 420 comprises determining a second pixel value of the second image of the pair of CT images, the second pixel value describing the value of a second pixel of the second image that depicts bone tissue of the subject, wherein the first pixel and the second pixel depict the same region of bone tissue of the subject

Once the first and second pixel values have been determined, the method proceeds to step 430 comprising determining, based on the determined first and second pixel values, a decomposition matrix of the pair of CT images for performing a material decomposition of the pair of CT images. In this exemplary method, step 430 comprises sub-steps 432, 434, and 436.

Step 432 comprises comparing the first pixel value to a plurality of precalculated pixel values each comprising a predicted value of a pixel that depicts bone tissue for a CT image having a different respective effective energy.

Step 434 comprises comparing the second pixel value to the plurality of precalculated pixel values.

Step 436 comprises determining the decomposition matrix of the pair of CT images based on the comparisons of steps 432 and 434.

Thus, an alternative method to that presented in Fig. 1, may involve pre-calculation of expected pixel values (or a range of expected pixel values) for CT images of different effective energy values. The fact that the value of the pixels depicting bone are known to lie on a predefined bone line in material space may permit these expected pixel values to easily be calculated for different effective energy values (e.g., by determining the transformation of the bone line 220 into energy space for different values of the effective energies of the pair of CT images). Therefore, by comparing the determined first and second pixel values to these precalculated expected pixel values or pixel value ranges an effective energy value for each of the pair of CT images may be calculated. The decomposition matrix may then be calculated from the determined effective energies.

Thus, in this exemplary embodiment the decomposition matrix is not determined directly from the first and second pixel values but involves the intermediate step of determining, based on the first and second pixel values, an effective energy value of each of the first and second images of the pair of CT images. The decomposition matrix is then determined based on the determined effective energy values of each of the first and second images of the pair of CT images. However, in alternative embodiments this need not be the case. The plurality of precalculated pixel values may in some embodiments comprise a plurality of pairs of pixel values calculated for a plurality of different decomposition matrices (rather than a plurality of different effective energies) and the decomposition matrix of the pair of CT images selected from the plurality of different decomposition matrices based on a comparison of the first and second pixel values to the plurality of pairs of pixel values.

Similarly, other aspects of the methods 100 and 400 may be implemented differently in alternative embodiments. In some embodiments the method may further comprise: determining a first body part of the subject depicted in the pixel of the first image of the pair of CT images associated with the first pixel value; determining a second body part of the subject depicted in the pixel of the second image of the pair of CT images associated with the second pixel value; and determining the decomposition matrix of the pair of CT images based on the first pixel value, the second pixel value, the first body part, and the second body part. The first and second body parts may comprise at least one of: a head; a thorax; an abdomen; a kidney region; a pelvis; and an extremity. Thus, information about the body part being imaged may be further used in the determination of the of the decomposition matrix to further improve the accuracy of the proposed method.

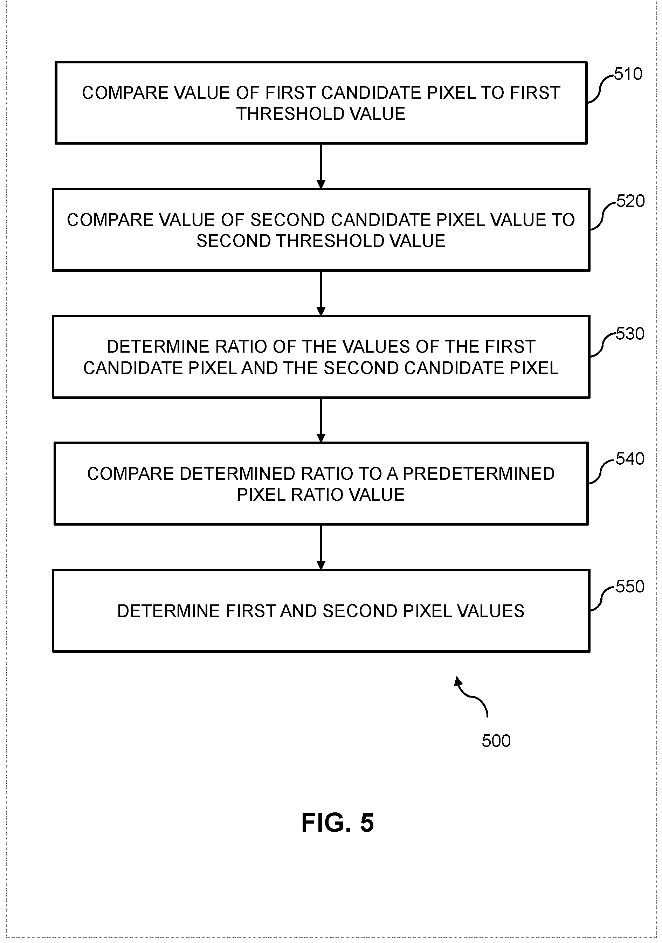

In certain embodiments of the proposed invention, identification of pixels of the first and second images of the pair of CT images may be achieved automatically without the requirement for the pixels depicting bone tissue of the subject to be manually identified by a clinician. Fig. 5 depicts an example of a method 500 for automatically identifying the first and second pixel values according to a proposed embodiment.

The method 500 commences with a step 510 comprising comparing the value of a first candidate pixel of the first image to a first threshold value. Regions of a CT image associated with bone tissue are likely to correspond to much higher pixel values than regions of the image depicting other biological tissues (see Fig. 2). Therefore, identifying the regions depicting bone tissue in the first image of the pair of CT images may first involve discarding from consideration all pixels whose values fall below a certain first threshold value.

Step 520 comprises comparing the value of the second candidate pixel of the second image to a second threshold value. Thus, a similar thresholding step may be carried out on the second image of the pair of CT images. The second threshold value may be the same as the first threshold value where no information about the scanning parameters is known but may be different from the first threshold value in an instance where information about the intensity and energy spectrum used to obtain the first and second images of the pair of CT images is known.

Once this initial thresholding has been carried out, the method 500 proceeds to step 530 comprising determining a pixel-wise ratio of the values of the first candidate pixel and the second candidate pixel. The method then proceeds to a step 540 comprising comparing the determined ratio of the value of the first candidate pixel to the value of the second candidate pixel to a predetermined pixel ratio value.

It was discussed with reference to Fig. 2, that it is expected that regions of bone tissue lie on a line in material space. This may also hold true in the image domain. Therefore, by determining the ratio of the first candidate pixel value to the second candidate pixel value, information may be garnered as to whether the pixel lies on the required line in image space for the pixel to be likely to depict bone tissue.

Therefore, determining the first and second pixel values may be achieved in a step 550 based on the comparisons made in steps 510, 520, 530, and 540.

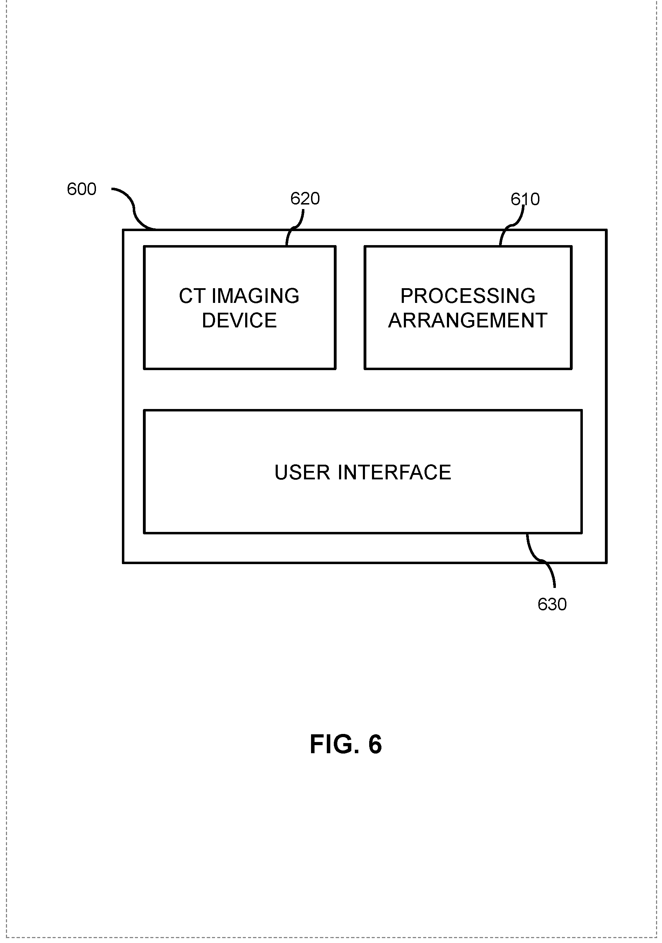

Referring now to Fig. 6, there is depicted a system 600 for determining the decomposition matrix of a pair of CT images of a subject.

The system 600 comprises a processing arrangement 610, a CT imaging device 620, and a user interface 630.

The CT imaging device 620 is configured to obtain a pair of CT images, wherein each image of the pair of CT images is obtained with a different incident energy spectrum. The CT imaging device 620 may be a dedicated spectral CT scanners such as dual-layer, fast kVp-switching, dual-source, and photon counting CT systems, or a conventional CT scanner equipped with slow kVp-switching.

Once the pair of CT images have been acquired they are passed to the processing arrangement 610. The processing arrangement is configured to: determine a first pixel value of a first image of the pair of CT images, the first pixel value describing the value of a first pixel of the first image that depicts bone tissue of the subject; determine a second pixel value of the second image of the pair of CT images, the second pixel value describing the value of a second pixel of the second image that depicts bone tissue of the subject, wherein the first and second pixels depict the same region of bone tissue of the subject; and determine, based on the determined first and second pixel values, a decomposition matrix of each of the first and second images of the pair of CT images.

The processing arrangement may then be further configured to carry out a material decomposition of the pair of CT images using the determined decomposition matrix. The result of this operation may then be presented to a clinician via the user interface 630.

The user interface 630 may further allow for a clinician to input various pieces of data such as patient data, or scan set-up data which may be used in the process of determining the decomposition matrix of the pair of CT images. For example, the user interface may be used to present the pair of CT images to the clinician and permit the clinician to select regions of the first and second images of the pair of CT images that depict bone tissue of the subject.

The system 600 shows how aspects of the proposed invention may be integrated into known CT scanning systems to improve their functionality.

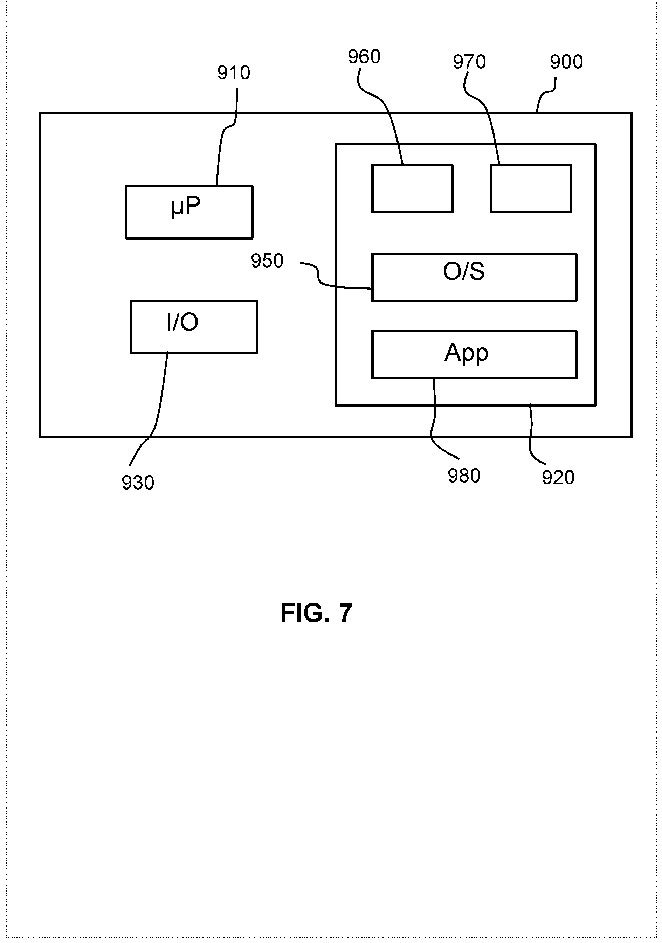

Fig. 7 illustrates an example of a computer 900 within which one or more parts of an embodiment may be employed. Various operations discussed above may utilize the capabilities of the computer 900. In this regard, it is to be understood that system functional blocks can run on a single computer or may be distributed over several computers and locations (e.g., connected via the internet).

The computer 900 includes, but is not limited to, PCs, workstations, laptops, PDAs, palm devices, servers, storages and the like. Generally, in terms of hardware architecture, the computer 900 may include one or more processors 910, memory 920 and one or more I/O device 930.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900.

The memory 920 can include any one or combination of volatile memory elements and non-volatile memory elements.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments.

The operating system 950 controls the execution of other computer programs, and provides scheduling, input-output control, file and data management, memory management, and communication control and related services. It is contemplated by the inventors that the application 980 for implementing exemplary embodiments, may be applicable on all commercially available operating systems.

Application 980 may be a source program, executable program (object code), script, or any other entity comprising a set of instructions to be performed.

When the computer 900 is in operation, the processor 910 is configured to execute software stored within the memory 920, to communicate data to and from the memory 920, and to generally control operations of the computer 900 pursuant to the software. The application 980 and the O/S 950 are read in whole or in part, by the processor 910, perhaps buffered within the processor 910, and then executed.

When the application 980 is implemented in software it should be noted that the application 908 can be stored on virtually any computer readable medium for use by or in connection with any computer related system or method. In the context of this document, a computer readable medium may be any electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device.

The methods of Figs 1, 4, and 5, and the system of Fig. 6, may be implemented in hardware or software, or a mixture of both (for example, as firmware running on hardware. To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a method as described above when the program is run on the one ore more physical computing devices.

To the extent that an embodiment is implemented partly or wholly in hardware, the blocks shown in the block diagrams of Figs 6 and 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, applications specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. IF a computer program is discussed above, it may be stored/distributed on a suitable medium, such as an optical storage medium or a solid state medium supplied together with or as part of other hardware, but may also be distributed in other forms, such as via the Internet or other wired or wireless telecommunication systems. If the term "adapted to" is used in the claims or description it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, with comprise one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in reverse order, depending upon the functionality involved. It will also be noted that each block of the block diagrams and/or flow diagrams, and combinations of blocks in the block diagrams and/or flow diagrams, can be implemented by special purpose hardware-based systems that perform the specified functions or acts or carry out combinations of special purpose hardware and computer instructions.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should be understood that the same reference numerals are used throughout the Figures to indicate the same or similar parts.

Implementations in accordance with the present disclosure relate to various techniques, methods, schemes and/or solutions pertaining to determining a decomposition matrix of a pair of CT images, wherein each of the pair of CT images is associated with a different energy spectrum. According to proposed concepts, several possible solutions may be implemented separately or jointly. That is, although these possible solutions may be described above separately, two or more of these possible solutions may be implemented in one combination or another.

**Claims**

1. A computer-implemented method (100) for determining a decomposition matrix of a pair of CT images of a subject, each image of the pair of CT images being associated with a different energy spectrum, the method comprising:

   determining (110) a first pixel value of a first image of the pair of CT images, the first pixel value describing the value of a first pixel of the first image that depicts bone tissue of the subject;
   determining (120) a second pixel value of the second image of the pair of CT images, the second pixel value describing the value of a second pixel of the second image that depicts bone tissue of the subject, wherein the first pixel and the second pixel depict the same region of bone tissue of the subject; and
   determining (130), based on the determined first and second pixel values, a decomposition matrix of the pair of CT images for performing a material decomposition of the pair of CT images.

2. The method of claim 1, wherein determining the decomposition matrix of the pair of CT images comprises:

   determining (132) a candidate decomposition matrix of the pair of CT images;
   performing (134), based on the determined candidate decomposition matrix, a material decomposition of the first and second pixel values to determine a predicted composition of the bone tissue of the subject;
   determining (138) an adjustment to be made to the candidate decomposition matrix based on the predicted composition of the bone tissue of the subject; and
   determining (130) the decomposition matrix of the pair of CT images based on the determined adjustment.

3. The method of claim 2, wherein the predicted composition of the bone tissue of the subject comprises a set of predicted coordinates of the bone tissue in a material space, and wherein determining the decomposition matrix of the pair of CT images further comprises:

   comparing (136) the set of predicted coordinates of the bone tissue in the material space to a predetermined line in material space; and
   determining (130) the decomposition matrix of the pair of CT images based on this comparison.

4. The method of claim 3, wherein the method further comprises:

   determining an anatomical feature associated with pixels of the first and second images associated with the first and second pixel values; and
   selecting the predetermined line in material space based on the determined anatomical feature.

5. The method of any of claims 2-4, wherein the predicted composition of the bone tissue comprises a set of predicted coordinates of the bone tissue in a material space comprising:

   a Compton effect component; and
   a photoelectric effect component.

6. The method of claim 1, wherein determining the first and second pixel values comprises:

   comparing (510) the value of a first candidate pixel of the first image to a first threshold value;
   comparing (520) the value of a second candidate pixel value of the second image to a second threshold value;
   determining (530) a ratio of the values of the first candidate pixel and the second candidate pixel;
   comparing (540) the determined ratio of the value of the first candidate pixel to the value of the second candidate pixel to a predetermined pixel ratio value; and
   determining (550) the first and second pixel values based these comparisons.

7. The method of claim 1, wherein determining the decomposition matrix of the pair of CT images comprises:

   comparing (432) the first pixel value to a plurality of precalculated pixel values each comprising a predicted value of a pixel that depicts bone tissue for a CT image having a different respective effective energy;
   comparing (434) the second pixel value to the plurality of precalculated pixel values; and
   determining (436) the decomposition matrix of the pair of CT images based on these comparisons.

8. The method of any preceding claim, wherein determining the decomposition matrix of the pair of CT images comprises:

   determining, based on the first and second pixel values, an effective energy value of each of the first and second images of the pair of CT images; and

   determining, based on the determined effective energy value of each of the first and second images, the decomposition matrix of the pair of CT images.

9. The method of any preceding claim, wherein the method further comprises:

   determining a first body part of the subject depicted in the pixel of the first image of the pair of CT images associated with the first pixel value;

   determining a second body part of the subject depicted in the pixel of the second image of the pair of CT images associated with the second pixel value; and

   determining the decomposition matrix of the pair of CT images based on the first pixel value, the second pixel value, the first body part and the second body part.

10. The method of claim 9, wherein the first and second body parts comprise at least one of:

    a head;
    a thorax;
    an abdomen;
    a kidney region;
    a pelvis; and
    an extremity.

11. The method of any preceding claims, wherein the method further comprises performing, before the first and second pixel values are determined, an image registration process of the first and second images of the pair of CT images such that the field of view of the first image is substantially identical to the field of view of the second image.

12. A computer program comprising computer program code means adapted, when said computer program is run on a computer, to implement the method of any of claims 1-11.

13. A system (600) for determining effective energies of a pair of CT images of a subject, each image of the pair of CT images being associated with a different energy spectrum, the system comprising:
    A processing arrangement (610) configured to:

    determine a first pixel value of a first image of the pair of CT images, the first pixel value describing the value of a first pixel of the first image that depicts bone tissue of the subject;

    determine a second pixel value of the second image of the pair of CT images, the second pixel value describing the value of a second pixel of the second image that depicts bone tissue of the subject, wherein the first and second pixels depict the same region of bone tissue of the subject; and

    determine, based on the determined first and second pixel values, an effective energy value of each of the first and second images of the pair of CT images.

14. The system of claim 13, wherein the system further comprises a CT imaging device (620) configured to obtain the pair of CT images, wherein each image of the pair of CT images is obtained with a different incident energy spectrum.

```
┌────────────────────────────────────────────────────┐
│           DETERMINE FIRST PIXEL VALUE                │ ⟋110
└────────────────────────────────────────────────────┘
                          │
                          ▼
┌────────────────────────────────────────────────────┐
│          DETERMINE SECOND PIXEL VALUE               │ ⟋120
└────────────────────────────────────────────────────┘
                          │
                          ▼
┌────────────────────────────────────────────────────┐
│         DETERMINE DECOMPOSITION MATRIX               │ ⟋130
│  ┌──────────────────────────────────────────────┐   │
│  │   DETERMINE CANDIDATE DECOMPOSITION MATRIX    │   │ ⟋132
│  └──────────────────────────────────────────────┘   │
│                      │                              │
│                      ▼                              │
│  ┌──────────────────────────────────────────────┐   │
│  │  PERFORM MATERIAL DECOMPOSITION OF FIRST AND  │   │ ⟋134
│  │         SECOND PIXEL VLAUES                   │   │
│  └──────────────────────────────────────────────┘   │
│                      │                              │
│                      ▼                              │
│  ┌──────────────────────────────────────────────┐   │
│  │  COMPARE SET OF PREDICTED COORDINATED TO      │   │ ⟋136
│  │  PREDETERMINED LINE IN MATERIAL SPACE         │   │
│  └──────────────────────────────────────────────┘   │
│                      │                              │
│                      ▼                              │
│  ┌──────────────────────────────────────────────┐   │
│  │  DETERMINE AN ADJUSTMENT TO BE TO THE         │   │ ⟋138
│  │  CANDIDATE DECOMPOSITION MATRIX               │   │
│  └──────────────────────────────────────────────┘   │
└────────────────────────────────────────────────────┘
                                              ↖100
```

**FIG. 1**

FIG. 2

FIG. 3A

FIG. 3B

DETERMINE FIRST PIXEL VALUE

410

DETERMINE SECOND PIXEL VALUE

420

DETERMINE DECOMPOSITION MATRIX

430

COMPARE FIRST PIXEL VALUE TO PLURALITY OF PRECALCULATED PIXEL VALUES

432

COMPARE SECOND PIXEL VALUE TO PLURALITY OF PRECALCULATED PIXEL VALUES

434

DETERMINE DECOMPOSITION MATRIX OF THE PAIR OF CT IMAGES

436

400

**FIG. 4**

COMPARE VALUE OF FIRST CANDIDATE PIXEL TO FIRST THRESHOLD VALUE — 510

COMPARE VALUE OF SECOND CANDIDATE PIXEL VALUE TO SECOND THRESHOLD VALUE — 520

DETERMINE RATIO OF THE VALUES OF THE FIRST CANDIDATE PIXEL AND THE SECOND CANDIDATE PIXEL — 530

COMPARE DETERMINED RATIO TO A PREDETERMINED PIXEL RATIO VALUE — 540

DETERMINE FIRST AND SECOND PIXEL VALUES — 550

500

# FIG. 5

600

620

610

CT IMAGING
DEVICE

PROCESSING
ARRANGEMENT

USER INTERFACE

630

**FIG. 6**

910   960   970   900

μP

950

O/S

I/O

App

930

980   920

# FIG. 7

Europäisches
Patentamt

European
Patent Office

Office européen
des brevets

# EUROPEAN SEARCH REPORT

**Application Number**

EP 24 21 7968

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/211338 A1 (KOJIMA SHINICHI [JP]) 7 July 2022 (2022-07-07) | 1,12,13 | INV. A61B6/00 |
| A | * abstract * * paragraph [0001] * * paragraph [0012] - paragraph [0013] * * paragraph [0033] * * paragraph [0035] - paragraph [0037] * * figures 10A, 11A * * paragraph [0048] * | 2-11,14 | A61B6/40 A61B6/42 A61B6/50 |
| X | JUMANAZAROV DONIYOR ET AL: "Material classification using basis material decomposition from spectral X-ray CT", NUCLEAR INST. AND METHODS IN PHYSICS RESEARCH, A, 22 August 2023 (2023-08-22), XP093271087, DOI: 10.5281/zenodo.4 | 1,12,13 | |
| A | * abstract * * section "2.5. Basis material decomposition and basis materials used" * * section "2.6. Calibration and calculations of the material property" * | 2-11,14 | |
| X | US 2008/226017 A1 (ALTMAN AMAIZ [IL] ET AL) 18 September 2008 (2008-09-18) | 1,12,13 | |
| A | * abstract * * paragraph [0001] * * paragraph [0063] - paragraph [0065] * * paragraph [0067] * * figures 3-5 * | 2-11,14 | |

**TECHNICAL FIELDS SEARCHED (IPC)**

A61B

The present search report has been drawn up for all claims

| Place of search | Date of completion of the search | Examiner |
|---|---|---|
| Munich | 16 April 2025 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 7968

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2025

| Patent document cited in search report | Publication date | Patent family member(s) | | Publication date |
|---|---|---|---|---|
| US 2022211338 A1 | 07-07-2022 | CN | 114720493 A | 08-07-2022 |
| | | JP | 7433256 B2 | 19-02-2024 |
| | | JP | 2022106241 A | 19-07-2022 |
| | | US | 2022211338 A1 | 07-07-2022 |
| US 2008226017 A1 | 18-09-2008 | CN | 101313332 A | 26-11-2008 |
| | | EP | 1927081 A2 | 04-06-2008 |
| | | JP | 5237812 B2 | 17-07-2013 |
| | | JP | 2009506854 A | 19-02-2009 |
| | | US | 2008226017 A1 | 18-09-2008 |
| | | WO | 2007029129 A2 | 15-03-2007 |

EPO FORM P0459

For more details about this annex : see Official Journal of the European Patent Office, No. 12/82